# FASCICULE DE BREVET EUROPEEN

(11) **EP 3 198 002 B1**
(45) Date de publication et mention de la délivrance du brevet: **09.09.2020**
(21) Numéro de dépôt: 15766181.0
(22) Date de dépôt: 21.09.2015
(51) Int. Cl.: C12N 1/20, C12N 15/72, C12P 21/02, C07K 14/47, C12R 1/19

(54) **SYSTEME D'EXPRESSION AUTO-INDUCTIBLE**
AUTOINDUZIERBARES EXPRESSIONSSYSTEM
AUTO-INDUCIBLE EXPRESSION SYSTEM

(30) Priorité: 24.09.2014 FR 1459019
(43) Date de publication de la demande: 02.08.2017
(73) Titulaire: Université de Bourgogne, 21078 Dijon Cedex (FR); INRA, 75338 Paris (FR); INSERM, 75654 Paris Cédex 13 (FR)
(72) Inventeur: NEIERS, Fabrice, F-21110 Pluvault (FR); SEIGNEURIC, Renaud, F-21110 Magny sur Tille (FR); BRIAND, Loïc, F-21850 Saint Apollinaire (FR); GARRIDO-FLEURY, Carmen, F-21240 Talant (FR)
(74) Mandataire: Oudin, Stéphane
(86) Numéro de dépôt international: PCT/EP2015/071619
(87) Numéro de publication internationale: WO 2016/046137

(56) Documents cités:
- OGANESYAN ET AL: "Effect of osmotic stress and heat shock in recombinant protein overexpression and crystallization", PROTEIN EXPRESSION AND PURIFICATION, ACADEMIC PRESS, SAN DIEGO, CA, vol. 52, no. 2, 27 janvier 2007 (2007-01-27), pages 280-285, XP005737467, ISSN: 1046-5928, DOI: 10.1016/J.PEP.2006.09.015
- LINDA L STEPHENS ET AL: "Co-expression of the molecular chaperone, Hsp70, improves the heterologous production of the antimalarial drug target GTP cyclohydrolase I,GCHI", PROTEIN EXPRESSION AND PURIFICATION, vol. 77, no. 2, 22 janvier 2011 (2011-01-22), pages 159-165, XP028176503, ISSN: 1046-5928, DOI: 10.1016/J.PEP.2011.01.005 [extrait le 2011-01-22]
- MARCION GUILLAUME ET AL: "C-terminal amino acids are essential for human heat shock protein 70 dimerization", CELL STRESS AND CHAPERONES, ALLEN PRESS ONLINE PUBLISHING, EDINBURGH, GB, vol. 20, no. 1, 17 juillet 2014 (2014-07-17), pages 61-72, XP035409489, ISSN: 1355-8145, DOI: 10.1007/S12192-014-0526-3 [extrait le 2014-07-17]
- NISHIHARA ET AL: "Chaperone Coexpression Plasmids: Differential and Synergistic Roles of DnaK-DnaJ-GrpE and GroEL-GroES in Assisting Folding of an Allergen of Japanese Cedar Pollen, Cryj2, in Escherichia coli", APPLIED AND ENVIRONMENTAL MICROBIOLOGY, AMERICAN SOCIETY FOR MICROBIOLOGY, US, vol. 64, no. 5, 1 mai 1998 (1998-05-01), pages 1694-1699, XP002135426, ISSN: 0099-2240
- LEE S C ET AL: "EFFECT OF OVERPRODUCTION OF HEAT SHOCK CHAPERONES GROESL AND DNAK ON HUMAN PROCOLLAGENASE PRODUCTION IN ESCHERICHIA COLI", JOURNAL OF BIOLOGICAL CHEMISTRY, AMERICAN SOCIETY FOR BIOCHEMISTRY AND MOLECULAR BIOLOGY, US, vol. 267, no. 5, 15 février 1992 (1992-02-15), pages 2849-2852, XP002029694, ISSN: 0021-9258
- STUDIER ET AL: "Protein production by auto-induction in high-density shaking cultures", PROTEIN EXPRESSION AND PURIFICATION, ACADEMIC PRESS, SAN DIEGO, CA, vol. 41, no. 1, 1 mai 2005 (2005-05-01), pages 207-234, XP027430000, ISSN: 1046-5928, DOI: 10.1016/J.PEP.2005.01.016 [extrait le 2005-03-28]
- L. BRIAND ET AL: "A self-inducible heterologous protein expression system in Escherichia coli", SCIENTIFIC REPORTS, vol. 6, no. 1, 9 September 2016 (2016-09-09), XP055458518, DOI: 10.1038/srep33037
- JINDAL S ET AL: "HUMAN STRESS PROTEIN HSP70: OVEREXPRESSION IN E.COLI, PURIFICATION AND CHARACTERIZATION", BIOTECHNOLOGY. THE INTERNATIONAL MONTHLY FOR INDUSTRIAL BIOLOGY, NATURE PUBLISHING GROUP, US, vol. 13, no. 10, 1 October 1995 (1995-10-01), pages 1105-1109, XP001247045, ISSN: 0733-222X, DOI: 10.1038/NBT1095-1105

## Description

### Domaine technique

La présente invention concerne notamment une bactérie E. Coli permettant l'expression à haut niveau d'une protéine d'intérêt. La présente invention concerne également un procédé de production d'une protéine d'intérêt mettant en œuvre une bactérie E. Coli selon l'invention. Par ailleurs, la présente invention concerne également un ensemble de parties permettant d'obtenir une bactérie E. Coli selon l'invention.

### Technique antérieure

La production de protéines recombinantes, comme les enzymes, les hormones et les anticorps, est nécessaire dans de nombreux domaines comme la recherche en biologie, la médecine humaine ou vétérinaire et l'industrie alimentaire. Cette production est souvent faite en cellules procaryotes et plus particulièrement en Escherichia coli (E. coli).

Le promoteur lac est le promoteur inductible le plus utilisé pour l'expression de protéine chez E. coli. Il peut être notamment utilisé pour induire l'expression de la T7 polymérase. Dans ce système, le gène codant la T7 polymérase est inséré dans le chromosome bactérien sous le contrôle de l'opéron lac. L'ajout d'isopropyle-β-D galactoside (IPTG) dans le milieu de culture permet d'induire efficacement la transcription de la T7 polymérase qui va ensuite pouvoir induire la transcription d'un gène, codant une protéine hétérologue, placé sous le contrôle d'un promoteur spécifique de la T7 polymérase.

Par exemple, Stephens et al. (Protein Expr Purif. 2011 Jun;77(2) :159-65) ont montré, qu'en présence d'IPTG, l'expression séquentielle de l'HSP70 de Plasmodium falciparum suivie de celle de la GMP cyclohydrolase I (PfGCHI) de Plasmodium falciparum augmentait l'expression de cette dernière.

Toutefois, l'IPTG présente des limitations pour la production industrielle de protéines recombinantes. Ces limitations sont notamment liées au coût et à la toxicité de l'IPTG. Par ailleurs, l'IPTG doit être introduit à un moment bien précis de la croissance bactérienne ce qui implique la nécessité de suivre précisément la densité cellulaire dans le milieu de culture.

Afin de contourner les problèmes liés à l'IPTG, différentes stratégies ont été mises en œuvre. Parmi celle-ci, on peut notamment citer l'utilisation de lactose ou du galactose comme inducteur de transcription. Mais ces derniers ne permettent pas d'atteindre des taux de production équivalents à l'IPTG et nécessitent toujours une surveillance précise de la croissance bactérienne.

Des systèmes auto-inductibles mettant en œuvre des milieux de culture spécifiques, comprenant des molécules métabolisées de telles sortes à induire automatiquement l'induction de la transcription à partir du promoteur lac, ont également été proposés.

Ces derniers systèmes, bien qu'efficaces, peuvent mettre en œuvre des molécules et des milieux qui ne sont pas encore approuvés par les autorités réglementaires. Il existe donc un besoin pour de nouvelles bactérie E. Coli capables d'exprimer de façon auto-inductible une protéine hétérologue sous le contrôle d'un promoteur lac dans un milieu de culture ne comprenant pas de molécules toxiques.

### Résumé de l'invention

Ainsi la présente invention concerne notamment un procédé pour l'expression d'une protéine d'intérêt par une bactérie E. Coli remarquable en ce qu'il comprend la culture d'une bactérie E. Coli exprimant de façon continue ou temporaire, sous le contrôle d'un promoteur lac, une protéine Hsp70 humaine, en ce que ladite bactérie E. Coli comprend en outre une séquence d'acide nucléique, codant une protéine d'intérêt, sous le contrôle d'un promoteur lac et en ce que ladite bactérie E. Coli est cultivée dans un milieu ne contenant pas d'IPTG.

La présente invention concerne également un procédé pour l'expression d'une protéine d'intérêt par une bactérie E. Coli remarquable en ce qu'il comprend la culture d'une bactérie exprimant de façon temporaire ou continue une protéine Hsp, en ce que ladite bactérie E. Coli comprend en outre une séquence d'acide nucléique, codant une protéine d'intérêt, sous le contrôle d'un promoteur lac et en ce que ladite bactérie E. Coli est cultivée dans un milieu ne contenant pas une molécule métabolisée de telle sorte à induire automatiquement l'induction de la transcription à partir du promoteur lac.

Dans le cadre de la présente invention, le terme « un milieu ne contenant pas une molécule métabolisée de telle sorte à induire automatiquement l'induction de la transcription à partir du promoteur lac » entend signifier que ledit milieu ne contient pas ladite molécule au moment de l'introduction desdites bactérie E. Coli et que ladite molécule n'est pas introduite, depuis l'extérieur, dans ledit milieu au cours du procédé selon l'invention.

Il a été observé, de façon surprenante, que l'expression d'une protéine Hsp70 humaine par la bactérie E. Coli induisait, même en l'absence d'IPTG, la transcription des séquences d'acides nucléiques sous le contrôle d'un promoteur lac. Bien que des milieux ou des conditions de culture particulières sans IPTG puissent conduire à l'induction de la transcription de ces séquences (voir par exemple Studier et al. protein expression and purification, academic press, vol. 41, 1, 2005, 207-234 ou le produit Overnight ExpressTM Autoinduction Systems de Novagen), la combinaison spécifique d'un promoteur lac et d'une protéine Hsp70 humaine permet d'obtenir cette induction tout en utilisant les milieux et les conditions de culture classiques.

Dans le cadre de la présente invention, le terme « promoteur lac » se réfère à un promoteur de l'opéron lac, dont l'activité de transcription est réprimée par une protéine répresseur comme la protéine LacI codée par le gène lacl mais levée par un inducteur, tel que le lactose ou des analogues de celui-ci (par exemple, l'isopropyle-β-D galactoside (IPTG)). L'inducteur se lie à la protéine répresseur et empêche la répression de la transcription génique.

Dans le cadre de la présente invention, le terme « sous le contrôle » entend signifier que la transcription de la séquence d'acide nucléique, codant ladite protéine d'intérêt, est dépendante de l'association d'une polymérase sur ledit promoteur. Avantageusement, ledit promoteur lac est placé en amont de ladite séquence d'acide nucléique, codant ladite protéine d'intérêt.

Selon un mode de réalisation préféré de l'invention, ladite protéine d'intérêt n'est pas la β-galactosidase, la lactose perméase et/ou la thiogalactoside transacétylase.

Selon un mode de réalisation préféré de l'invention ladite protéine d'intérêt est une protéine hétérologue.

Dans le cadre de la présente invention, le terme « protéine hétérologue » entend signifier que ladite protéine n'est pas une protéine naturellement exprimée par ladite bactérie E. Coli.

Selon un mode de réalisation préféré, ledit acide nucléique codant une protéine d'intérêt est compris dans un premier plasmide. Dans le cadre de ce mode de réalisation, ledit plasmide comprend le promoteur lac.

Selon un mode de réalisation préféré, ladite protéine d'intérêt est une protéine recombinante et de façon encore plus préféré une protéine de mammifère et de façon tout à fait préféré une protéine humaine. Selon un mode de réalisation encore plus préféré ladite protéine recombinante n'est pas une polymérase.

Selon un autre mode de réalisation préféré, ladite protéine d'intérêt est une polymérase. Selon un mode de réalisation encore plus préféré, ladite polymérase est une T7 polymérase.

Dans le cadre de ces deux derniers modes de réalisations, la bactérie E. Coli selon l'invention comprend en outre une deuxième séquence d'acide nucléique, codant une seconde protéine d'intérêt placé sous le contrôle d'un promoteur spécifique de ladite polymérase. Ainsi, dans ce mode de réalisation c'est l'induction de l'expression de la polymérase qui va permettre l'expression de la protéine d'intérêt. Selon un mode de réalisation préféré ladite deuxième séquence d'acides nucléiques est comprise dans ledit premier plasmide ou dans un second plasmide. Selon un mode de réalisation encore plus préféré, ladite séquence codant pour la polymérase sous le contrôle d'un promoteur Lac est comprise dans le génome de ladite bactérie E. Coli.

Selon un mode de réalisation préféré, ladite protéine Hsp est codée par une séquence d'acides nucléiques sous le contrôle d'un promoteur lac.

Selon un mode de réalisation préféré, ladite bactérie E. Coli appartient à la souche BL21(DE3) star ou à la souche BL21 (DE3) .

Dans le cadre de la présente invention, le terme « protéine Hsp » fait référence aux « heat shock protein » c.à.d. aux protéines appartenant au groupe de protéines dont l'expression est naturellement induite par un choc thermique. Ces protéines sont bien connues de l'homme du métier. Elles sont notamment impliquées dans le pliage et le dépliage des autres protéines. Leur expression est augmentée lorsque les cellules sont exposées à des températures élevées ou à d'autres stress. Les Hsp sont trouvées dans presque tous les organismes vivants, de la bactérie à l'homme. Les protéines de choc thermique sont nommées en fonction de leur poids moléculaire. Par exemple, la Hsp60, Hsp70 et Hsp90 se réfèrent à des familles de protéines de choc thermique de l'ordre de 60, 70 et 90 kilodaltons par la taille, respectivement.

Dans le cadre de la présente invention, le terme « protéine Hsp » fait référence aux protéines Hsp produites naturellement par les cellules eucaryotes ou procaryotes. Le terme « protéine Hsp » fait également référence aux protéines présentant une séquence ayant une homologie supérieure à 90%, préférentiellement supérieure à 95% et tout à fait préférentiellement supérieure à 99% aux protéines Hsp naturellement produites. Dans le cadre de la présente invention, est considérée comme une séquence protéique, homologue d'une autre séquence protéique, une protéine qui présente, sur un nombre d'acides aminés au moins égal à 80 pourcent du nombre d'acides aminés de la séquence protéique de référence, de préférence sur la longueur totale de la séquence de référence, un pourcentage d'homologie correspondant aux valeurs indiquées précédemment. Le pourcentage d'homologie est calculé en comptant le nombre de positions pour lesquelles les séquences protéiques présentent des acides aminés identiques et en divisant ce nombre de positions identiques par la longueur de la séquence du polypeptide le plus long et en multipliant le résultat obtenu par 100 pour obtenir le pourcentage d'homologie entre ces deux séquences.

La protéine Hsp est la protéine Hsp70 humaine (Uniprot reference number : P08107).

Selon un mode de réalisation préféré, ladite bactérie E. Coli est cultivée dans un milieu liquide.

De façon surprenante, la demanderesse a observé que la concentration en glucose et/ou en lactose du milieu de culture utilisé permet de moduler le moment où la protéine d'intérêt est exprimée par la bactérie E. Coli selon l'invention.

Ainsi, selon un mode de réalisation préféré de l'invention, le milieu de culture comprend du glucose. Selon un mode de réalisation encore plus préféré ledit milieu de culture comprend entre 0.01% et 0.5% de glucose et selon un mode de réalisation tout à fait préféré entre 0.3% et 0.7% de glucose.

Ainsi, selon un autre mode de réalisation préféré de l'invention, le milieu de culture comprend du lactose. Selon un mode de réalisation encore plus préféré ledit milieu de culture comprend entre 0.01% et 0.5% de lactose et selon un mode de réalisation tout à fait préféré entre 0.3% et 0.7% de lactose.

### Description des modes de réalisation

### Expression de protéines hétérologues par une bactérie exprimant l'Hsp70 humaine

### Matériels et méthodes

Dans toutes les expériences suivantes une souche de bactérie E. coli BL21(DE3)star a été utilisée. Toutefois, l'invention n'est pas limitée à ce type particulier de bactérie E. Coli.

La séquence codant l'Hsp70 humaine a été clonée dans le plasmide d'expression bactérien pET21d et placée sous le contrôle du promoteur T71ac. Ce plasmide contient par ailleurs un gène de résistance à l'ampicilline.

Le plasmide pET21d-Hsp70 a été utilisé pour transformer E. coli BL21(DE3)Star et obtenir une bactérie capable d'exprimer l'Hsp70.

Un second plasmide pET comprenant un gène de résistance à la kanamycine et la même origine de réplication que le plasmide pET21d a été utilisé pour cloner un gène codant pour une protéine hétérologue. Ce second plasmide a été utilisé pour transformer la souche bactérienne codant Hsp70.

La production de cinq protéines hétérologues différentes a été testée, ces protéines sont :
- La méthionine sulfoxyde réductase B (MsrB) de Xanthomonas compestris.
- La thioredoxine 1 (Trx1) d'E. coli.
- La purine nucléoside phosphorylase (PNP) d'E. coli.
- Le domaine N-Terminal du récepteur T1R3 d'Homo sapiens.
- La miraculine de Richardella dulcifica (MCL).

Ces cinq protéines sont d'origines différentes (bactérie, plante ou homme) et de taille variant de 14kDa à 45kDa. Elles présentent par ailleurs différentes fonctions (enzyme, récepteur ou ligand) et localisation cellulaire (cytoplasme, plasma ou membrane).

Les bactérie E. Coli transformées ont été cultivées en milieu LB, dans des volumes différents (5ml, 20 ml, 1L) à différentes températures (25°C, 30°c et 37°C).

Les milieux de culture obtenus ont été analysés par électrophorèse PAGE-SDS.

### Résultats

Toutes les protéines testées sont exprimées à haut niveau à 37°C en l'absence d'inducteur. Ainsi on peut observer sur les électrophorèses PAGE-SDS que la protéine hétérologue est la protéine la plus fortement exprimée par la bactérie.

Une accumulation des protéines hétérologues est observée pendant au moins 20 heures.

La production des protéines hétérologues n'est pas dépendante du volume de milieu de culture utilisé.

Ces résultats sont retrouvés à toutes les températures compatibles avec la croissance bactérienne.

Le procédé selon l'invention a été mis en œuvre, avec le même succès, avec des protéines hétérologues de mammifères (notamment humaines), de bactérie E. Coli et de plantes. Parmi celles-ci, on peut notamment citer les GST humaines (GSTA1 et GSTP1), de drosophile (GSTD2 et GSTD7), une lipocaline humaine (LCN1), et l'Hsp110 humaine.

Le procédé selon l'invention fonctionne également quelque soit la localisation de la protéine : membranaire, extracellulaire ou cytoplasmique.

Le procédé selon l'invention n'est pas dépendant de la structure du plasmide utilisé et a pu être mis en œuvre avec succès avec les plasmides pD431-SR, pD451-SR, pD434-SR, pD434-WR, pD454-WR, pJ431:2047, pJ414:2047 (DNA2.0 Menlo Park, USA) .

### Effets du glucose et du lactose sur l'expression des protéines d'intérêts

### Matériels et méthodes

Les bactérie E. Coli transformées pour exprimer les protéines recombinantes citées précédemment ont été cultivées en milieu LB supplémenté avec 0.05% de glucose ou 0.2% de lactose.

La croissance bactérienne a été suivie en mesurant la densité optique à 600nm et la quantité de protéine recombinante produite a été mesurée par chromatographie PAGE-SDS du surnageant de culture.

### Résultats

En l'absence de glucose ou de lactose, l'expression des protéines d'intérêt apparait à une densité optique de 1.

L'ajout de 0.05% de glucose dans le milieu retarde l'expression de la protéine d'intérêt à une densité optique à 600nm de 1.5.

L'ajout de 0.02% de lactose permet d'accélérer la production de la protéine d'intérêt à une densité optique de 0.7.

## Revendications

1. Procédé pour l'expression d'une protéine d'intérêt par une bactérie Escherichia coli (E. coli) **caractérisé en ce qu'**il comprend la culture d'une bactérie exprimant de façon continue ou temporaire, sous le contrôle d'un promoteur lac, une protéine Hsp70 humaine **en ce que** ladite bactérie E. coli comprend en outre une séquence d'acide nucléique, codant une protéine d'intérêt, sous le contrôle d'un promoteur lac et **en ce que** ladite bactérie E. coli est cultivée dans un milieu ne contenant pas d'IPTG ou une molécule métabolisée de telle sorte à induire automatiquement l'induction de la transcription à partir du promoteur lac.

2. Procédé selon la revendication précédente **caractérisé en ce que** ledit acide nucléique codant une protéine d'intérêt est compris dans un premier plasmide.

3. Procédé selon l'une des revendications précédentes **caractérisé en ce que** ladite protéine d'intérêt est une protéine recombinante.

4. Procédé selon l'une des revendications 1 à 2 **caractérisé en ce que** ladite protéine d'intérêt est une polymérase.

5. Procédé selon la revendication précédente **caractérisé en ce que** ladite polymérase est une T7 polymérase.

6. Procédé selon l'une des revendications 4 à 5 **caractérisé en ce que** ladite bactérie E. coli comprend en outre une deuxième séquence d'acide nucléique, codant une seconde protéine d'intérêt placé sous le contrôle d'un promoteur spécifique de ladite polymérase.

7. Procédé selon la revendication précédente **caractérisé en ce que** ladite deuxième séquence d'acides nucléiques est comprise dans ledit premier plasmide ou dans un second plasmide.

8. Procédé selon l'une des revendications 1 à 7 **caractérisé en ce que** ladite bactérie E. coli appartient à la souche BL21(DE3) ou BL21(DE3)star.

9. Procédé selon l'une des revendications précédentes **caractérisé en ce que** ladite bactérie E. coli est cultivée dans un milieu liquide.

## Patentansprüche

1. Verfahren für die Expression eines Proteins von Interesse durch ein Bakterium Escherichia coli (E. coli), **dadurch gekennzeichnet, dass** es die Kultur eines Bakteriums umfasst, das kontinuierlich oder temporär, unter der Kontrolle eines lac-Promotors, ein menschliches Hsp70-Protein exprimiert, dadurch, dass das Bakterium E. coli des Weiteren eine Nukleinsäuresequenz umfasst, die ein Protein von Interesse codiert, unter der Kontrolle eines lac-Promotors, und dadurch, dass das Bakterium E. coli in einem Medium kultiviert wird, das kein IPTG oder ein metabolisiertes Molekül enthält, sodass die Induktion der Transkription ausgehend von dem lac-Promotor automatisch induziert wird.

2. Verfahren nach dem vorstehenden Anspruch, **dadurch gekennzeichnet, dass** die Nukleinsäure, die ein Protein von Interesse codiert, in einem ersten Plasmid enthalten ist.

3. Verfahren nach einem der vorstehenden Ansprüche, **dadurch gekennzeichnet, dass** das Protein von Interesse ein rekombinantes Protein ist.

4. Verfahren nach einem der Ansprüche 1 bis 2, **dadurch gekennzeichnet, dass** das Protein von Interesse eine Polymerase ist.

5. Verfahren nach dem vorstehenden Anspruch, **dadurch gekennzeichnet, dass** die Polymerase eine T7-Polymerase ist.

6. Verfahren nach einem der Ansprüche 4 bis 5, **dadurch gekennzeichnet, dass** das Bakterium E. coli des Weiteren eine zweite Nukleinsäuresequenz umfasst, die ein zweites Protein von Interesse codiert, platziert unter der Kontrolle eines spezifischen Promotors der Polymerase.

7. Verfahren nach dem vorstehenden Anspruch, **dadurch gekennzeichnet, dass** die zweite Nukleinsäuresequenz in dem ersten Plasmid oder in einem zweiten Plasmid enthalten ist.

8. Verfahren nach einem der Ansprüche 1 bis 7, **dadurch gekennzeichnet, dass** das Bakterium E. coli zu dem Stamm BL21(DE3) oder BL21(DE3) Star gehört.

9. Verfahren nach einem der vorstehenden Ansprüche, **dadurch gekennzeichnet, dass** das Bakterium E. coli in einem flüssigen Medium kultiviert wird.

## Claims

1. Method for the expression of a protein of interest by an Escherichia coli (E. coli) bacterium **characterised in that** it comprises the culturing of a bacterium continuously or temporarily expressing, under the control of a lac promoter, a human Hsp70 protein, **in that** said E. coli bacterium further comprises a nucleic acid sequence, encoding a protein of interest, under the control of a lac promoter and **in that** said E. coli bacterium is cultured in a medium not containing IPTG or a metabolised molecule in such a way as to automatically induce the induction of transcription from the lac promoter.

2. Method according to the preceding claim **characterised in that** said nucleic acid encoding a protein of interest is comprised in a first plasmid.

3. Method according to one of the preceding claims **characterised in that** said protein of interest is a recombinant protein.

4. Method according to one of claims 1 to 2 **characterised in that** said protein of interest is a polymerase.

5. Method according to the preceding claim **characterised in that** said polymerase is a T7 polymerase.

6. Method according to one of claims 4 to 5 **characterised in that** said E. coli bacterium further comprises a second nucleic acid sequence, encoding a second protein of interest placed under the control of a specific promoter of said polymerase.

7. Method according to the preceding claim **characterised in that** said second nucleic acid sequence is comprised in said first plasmid or in a second plasmid.

8. Method according to one of claims 1 to 7 **characterised in that** said E. coli bacterium belongs to the BL21(DE3) or BL21(DE3)star strain.

9. Method according to one of the preceding claims **characterised in that** said E. coli bacterium is cultured in a liquid medium.
